# EUROPÄISCHE PATENTSCHRIFT

(11) **EP 0 306 813 B1**
(45) Veröffentlichungstag und Bekanntmachung des Hinweises auf die Patenterteilung: **01.06.1994**
(21) Anmeldenummer: 88114180.8
(22) Anmeldetag: 31.08.1988
(51) Int. Cl.: G01N 33/86

(54) **Verfahren zur Bestimmung von "löslichem" Fibrin**
Method for the determination of soluble fibrin
Procédé de déterminer de la fibrine soluble

(30) Priorität: 08.09.1987 DE 3730059
(43) Veröffentlichungstag der Anmeldung: 15.03.1989
(73) Patentinhaber: BEHRINGWERKE Aktiengesellschaft, 35001 Marburg (DE)
(72) Erfinder: Becker, Udo, Dr., D-3550 Marburg (DE); Braun, Konrad, D-3557 Ebsdorfergrund (DE); Heimburger, Norbert, Prof. Dr., D-3550 Marburg (DE)

(56) Entgegenhaltungen:
- PROCEEDINGS OF THE NATL. ACADEMY OF SCIENCES USA, Band 82, Oktober 1985, Washington, DC (US); U. SCHEEFERS-BORCHEL et al., Seiten 7091-7095#
- CHEMICAL ABSTRACTS, Band 90, Nr. 11, 12 März 1979, Columbus, OH (US); A. BINI et al., Seite 252, Nr. 83069m#
- CHEMICAL ABSTRACTS, Band 93, Nr. 3, 21 Juli 1980, Columbus, OH (US); P.K. LUND et al., Seite 317, Nr. 21837p#
- CLINICAL CHEMISTRY. Band 31, Nr. 9, September 1985, Winston (US); R.S. SCHIFREEN et al., Seiten 1468-1473#

## Beschreibung

Die Erfindung betrifft ein Verfahren zur Bestimmung von "löslichem" Fibrin in einer Körperflüssigkeit einer Spezies, unter Verwendung eines an eine Festphase gebundenen bioaffinen Bindungspartners für dieses Fibrin und eines markierten anderen bioaffinen Bindungspartners für dieses Fibrin, wobei dieser andere Bindungspartner so ausgewählt ist, daß er mit dem ersten nicht kreuzreagiert. Als Bindungspartner werden Fibrinogen und Antikörper verwendet.

In der klinischen Diagnostik ist der Nachweis einer Aktivierung des Gerinnungssystems von Wichtigkeit. So kann zum Beispiel nach einer Operation eine Aktivierung der Gerinnung eintreten, die zu einer Thrombose führt. Eine Thrombose ist ein Ereignis, für deren Vorhersage es bisher keine zuverlässigen diagnostischen Kriterien gibt.

In allen Fällen ist anzunehmen, daß vor diesem Ereignis eine latent ablaufende Gerinnung bereits stattfindet, die aber durch gegenläufige Regulation des Organismus (z.B. durch fibrinolytische Prozesse) noch unter Kontrolle gehalten wird. In dieser Phase besteht ein Bedürfnis nach diagnostischen Kriterien zur Erkennung dieser bedrohlichen Situation.

Ein möglicher Meßparameter ist das Fibrin, das aus Fibrinogen durch Einwirkung von Thrombin gebildet wird. Fibrin ist das Material, das bei einer Thrombose den Gefäßverschluß verursacht. In niedrigen Konzentrationen, wie es in der oben beschriebenen Phase des "präthrombotischen Zustand" vorkommt, ist es jedoch löslich und kann durch geeignete Nachweisverfahren zur Diagnose eines präthrombotischen Zustands herangezogen werden.

Die gültige Vorstellung ist, daß Fibrin sich an Fibrinogen anlagert und dadurch in Lösung gehalten wird. Wird ein bestimmtes Verhältnis von Fibrin zu Fibrinogen überschritten, so bildet sich unlösliches Fibrin, das Material für den thrombotischen Gefäßverschluß.

Aufgabe eines Verfahren zum Nachweis löslichen Fibrins ist es daher, Fibrin in Anwesenheit eines mehr oder weniger großen Überschusses an Fibrinogen spezifisch nachzuweisen.

Im allgemeinen macht man sich dabei Methoden zunutze, die die Löslichkeit des Fibrins herabsetzen: Gebräuchlich ist zum Beispiel der sogenannte Äthanol-Gelierungstest nach Breen und Tullis (Ann.Intern.Med. 69, 1197-1206, 1986) und der Protaminsulfattest nach Niewiarowski und Gurewich (J.Lab.Clin.Med. 77, 665-676, 1971). Neuere Methoden bedienen sich der Eigenschaft des Fibrins, die Wirkung der Plasminogen-Aktivatoren zu verstärken (B. Wimann und M. Ranby, Thromb.Haemostas. 55, 189-193, 1986). In einer anderen Näherungsweise wurden Antikörper erzeugt, die die geringen strukturellen Unterschiede zwischen Fibrinogen und Fibrin erkennen können (U. Scheefers-Borchel et al., Proc.Natl.Acad.Sci. 82, 7091-7095, 1985).

In Chemical Abstracts Vol. 93 (1980), 21837p ist ein Verfahren zur Bestimmung von löslichem Fibrin beschrieben, bei der das lösliche Fibrin zunächst durch Chromatographie an einer Fibrinogen-Sepharose Säule isoliert wird. Ein ähnliches Verfahren wird zur Fibringewinnung in Chemical Abstracts Vol. 90 (1979), 83039m beschrieben.

Alle genannten Verfahren haben mehr oder weniger große Nachteile, die teils in der zu geringen Empfindlichkeit und teils in der nicht ausreichenden Spezifität begründet oder auch in der klinischen Routine zu aufwendig sind.

Überraschenderweise wurde gefunden, daß von der bekannten Eigenschaft des Fibrins an Fibrinogen zu binden, Gebrauch gemacht werden kann, um ein neues und überraschend einfaches Verfahren zur Bestimmung von löslichem Fibrin zur Verfügung zu stellen. Es verbindet eine funktionelle Eigenschaft des Fibrins, nämlich die der spezifischen Bindung an Fibrinogen mit einem immunologischen Verfahren, ohne daß dazu ein spezieller spezifischer Antikörper gegen Fibrin erforderlich ist.

Gegenstand der Erfindung ist daher ein Verfahren zur Bestimmung von "löslichem" Fibrin in einer Körperflüssigkeit einer Spezies, wobei eine Probe dieser Körperflüssigkeit entweder
- mit einem an eine Festphase gebundenen Fibrinogen in Kontakt gebracht wird,
- die Körperflüssigkeit abgetrennt wird,
- die Festphase mit einem markierten Antikörper, der mit Fibrin reagiert, in Kontakt gebracht wird,
- der Überschuß dieses Antikörpers abgetrennt wird und aus der Menge der an die Festphase gebundenen Markierung die Menge "löslichen" Fibrins bestimmt wird
oder
- mit einem an eine Festphase gebundenen Antikörper, der mit Fibrin reagiert, in Kontakt gebracht wird,
- die Körperflüssigkeit abgetrennt wird,
- die Festphase mit einem markierten Fibrinogen in Kontakt gebracht wird,
- der Überschuß dieses Fibrinogens abgetrennt wird und aus der Menge der an die Festphase gebundenen Markierung die Menge "löslichen" Fibrins bestimmt wird
dadurch gekennzeichnet, daß das Fibrinogen und der Antikörper aus der gleichen Tierspezies gewonnen wurden, die verschieden ist von der Spezies von der die Körperflüssigkeit stammt.

Vorzugsweise stammt die Körperflüssigkeit von der Spezies Mensch.

Es ist zweckmäßig, Fibrinogen und Antikörper so zu wählen, daß das Fibrinogen ein Fibrinogen einer anderen Spezies ist als das, aus der die Körperflüssigkeit stammt, und der Antikörper ein gegen Fibrin gerichteter Antikörper aus dieser anderen Spezies ist, um die Möglichkeit einer Kreuzreaktion zu vermindern.

Falls an die Festphase ein Fibrinogen einer anderen Spezies als der, aus der die Körperflüssigkeit stammt, gebunden ist, kann die Körperflüssigkeit in Gegenwart von zusätzlichem Fibrinogen dieser anderen Spezies mit der Festphase in Kontakt gebracht werden.

Als Markierung ist besonders ein Enzym geeignet.

Gegenstand der Erfindung ist weiterhin die Verwendung von Fibrinogen einer Art zur Bestimmung von "löslichem" Fibrin in einer Körperflüssigkeit einer anderen Art. Eine bevorzugte Ausführungsform verwendet Fibrinogen, das an einen festen Träger gebunden ist. Als fester Träger kann eine Kunststoffoberfläche, wie sie für die Festphasen-Immuntests (ELISA) verwendet werden, dienen, zum Beispiel Mikrotitrationsplatten, Polystyrolröhrchen oder Kunststoffkugeln. Die Beschichtung mit Fibrinogen kann sowohl adsorptiv als auch kovalent erfolgen. Im besonderen sind Polystyroloberflächen geeignet, die durch Imprägnierung mit einer Fibrinogenlösung beschichtet werden können. Die für die Beschichtung einzusetzende Fibrinogenlösung wird im allgemeinen in einer Konzentration von mehr als 1 mg/l angewendet, wobei die exakte Konzentration nicht entscheidend ist, da die Adsorption mehr von der Bindungkapazität der Oberfläche als von der Konzentration der Lösung abhängt. Das für die Beschichtung einzusetzende Fibrinogen darf mit dem im Test verwendeten Antikörper gegen Fibrin nicht kreuzreagieren. Am einfachsten wird dies dadurch erreicht, daß Fibrinogen derselben Spezies verwendet wird, die für die Herstellung der markierten Antikörper gegen das zu bestimmende Fibrin dient. Solchermaßen mit Fibrinogen beschichtete Oberflächen sind nun in der Lage, "lösliches" Fibrin aus einer eingebrachten Körperflüssigkeit zu adsorbieren. Die Flüssigkeit wird abgetrennt und die Oberfläche gewaschen. Nun kann mit einem Antikörper das aus der Probe heraus adsorbierte Fibrin nachgewiesen werden. Die Eigenschaften dieses Antikörpers sind nun so auszuwählen, daß er mit dem Fibrin der Probe reagiert, nicht aber mit dem für die Beschichtung der Kunststoffoberfläche verwendeten Fibrinogen. Wenn man lösliches Fibrin in menschlichem Blutplasma bestimmen will, so ist ein Antikörper gegen Humanfibrinogen oder gegen dessen Spaltprodukte geeignet. Es ist unwesentlich, ob es sich um einen polyklonalen oder monoklonalen Antikörper handelt. Wichtig ist nur, daß er nicht mit dem Fibrinogen, das für die Beschichtung der Festphase verwendet wurde, kreuzreagiert. Am einfachsten wird diese Bedingung dadurch erfüllt, daß Antikörper und Beschichtungsfibrinogen von derselben Spezies verwendet werden, da gegen körpereigene Proteine keine Antikörper gebildet werden. Die Antikörper können markiert sein, besonders mit einem Radioisotop oder vorzugsweise einem Enzym, um in bekannter Weise den Bindungnachweis zu führen. Ebenso kann in einer nachfolgenden Reaktion ein markierter Antikörper gegen den ersten Antikörper ("Sandwich-Assay") verwendet werden.

In einer speziellen Ausführungsform kann dem Inkubationsmedium Fibrinogen zugesetzt werden. Damit werden unterschiedliche Fibrinogengehalte der Untersuchungsproben ausgeglichen. Da lösliches Fibrin durch Fibrinogen in Lösung gehalten wird, ist denkbar, daß unterschiedliche Gehalte von Fibrinogen in der Probe einen Einfluß auf das Meßergebnis haben könnten. Dieser Einfluß kann daher durch Hinzufügen von Fibrinogen aufgehoben werden. Es ist nicht zweckmäßig hierzu Humanfibrinogen zu verwenden, wenn lösliches Humanfibrin bestimmt werden soll, da gereinigtes Humanfibrinogen Spuren von löslichem Fibrin enthalten kann, was eine Störung des Nachweisverfahrens bedeuten würde. Zweckmäßig ist daher die Verwendung von Fibrinogen der Tierart, die zur Gewinnung des verwendeten Antikörpers dient, zum Beispiel Kaninchenfibrinogen, um bei vorhandener Verunreinigung des Fibrinogens mit löslichem Fibrin keine Kreuzreaktion mit Anti-Fibrinogen zu erhalten.

Die Erfindung wird durch nachfolgende Beispiele erläutert.

### Beispiel 1

### Beschichtung von Polystyrolröhrchen mit Kaninchen-Fibrinogen

Kaninchen-Fibrinogen (Herstellung nach R.M. Huseby und N.Bang: Fibrinogen, in Thrombosis and Bleeding Disorders, Thieme-Verlag Stuttgart, N.Bang, F.Beller, E.Deutsch und E.Mammen (Eds.) S. 222-247, 1971) 20 mg/l wurde in 0,01 mol/l Phosphatpuffer, pH 7,4 gelöst und je 0,25 ml in Polystyrolröhrchen (Fa. Greiner) gefüllt. Nach Inkubation über Nacht wurde mit 0,05 mol/l Tris/Zitronensäure-Puffer, pH 7,4 mehrmals gewaschen und die Röhrchen im Trockenschrank über Kieselgel bei Raumtemperatur getrocknet. Nach dem Trocknen wurden sie in Aluminiumbeutel bis zur späteren Verwendung luft- und feuchtigkeitsdicht verschlossen.

### Beispiel 2

### Herstellung eines Konjugats aus Anti-Humanfibrinogen vom Kaninchen und Peroxidase

Antiserum gegen Humanfibrinogen vom Kaninchen (Behringwerke, Produkt-Nr. ORCH) wurde mit dem gleichen Volumen gesättigter Ammoniumsulfatlösung gemischt und das Präzipitat abzentrifugiert. Nach zweimaligem Waschen mit 50%iger Ammoniumsulfatlösung wurde das Präzipitat in wenig 0,2 mol/l Na-Phosphat, pH 8,2 gelöst und gegen denselben Puffer dialysiert. Diese Antikörperlösung wurde dann mit Peroxidase (Boehringer Mannheim, Bestell-Nr. 108090) in bekannter Weise gekuppelt. Vorschrift siehe S. Avrameas, Immunochemistry 6, 43 (1969). Das so hergestellte Konjugat wurde auf eine für den Test geeignete Verdünnung vorverdünnt und portionsweise bei -70°C eingefroren.

### Beispiel 3

### Erzeugung von Blutplasma mit einem Gehalt an löslichem Fibrin

Human-Citratplasma, zum Beispiel Standard-Human-Plasma (Behringwerke, Bestell-Nr. ORKL) wurde mit 0,05 IU/ml Thrombin, zum Beispiel Testthrombin (Behringwerke, Bestell-Nr. ORHT) versetzt und nach bestimmten Zeiten 0,2 ml Aliquote zum Abstoppen der Thrombinreaktion in 0,02 ml einer Mischung aus Hirudin 10 E/ml und Heparin 20 E/ml pipettiert. Als Vergleich diente eine Plasmaprobe, die mit physiologischer Kochsalzlösung anstelle von Thrombin versetzt wurde.

### Beispiel 4

### Durchführung eines Tests auf lösliches Fibrin

Die nach Beispiel 3 hergestellten Proben wurden vor dem Test mit einem Tris-Puffer (0,05 mol/l, pH 7,4, enthaltend ^{R}Tween 80 0,5%, EDTA 5 mmol/l und Antagosan 70 KIE/ml) 1:100 verdünnt. In die nach Beispiel 1 hergestellten, mit Kaninchen-Fibrinogen beschichteten Röhrchen wurden nacheinander 0,2 ml der vorverdünnten Proben pipettiert und 2 Stunden bei Raumtemperatur inkubiert. Anschließend wurde abgesaugt und dreimal mit Puffer (z.B. Enzygnost-Waschpuffer der Behringwerke, Bestell-Nr. OSNK) gewaschen.
Es wurde nun in jedes Röhrchen je 0,2 ml des nach Beispiel 2 hergestellten Antikörper-Konjugats pipettiert und 2 Stunden bei Raumtemperatur inkubiert. Es wurde wiederum dreimal gewaschen und je 0,2 ml Substratlösung (z.B. Substratlösung O-Phenylendiamin, Behringwerke, Bestell-Nr. OSNK) eingefüllt. Nach 10 min bei Raumtemperatur wurde die Reaktion mit je 1 ml 2 normale Schwefelsäure abgestoppt und die Extinktion der Proben bei 492 nm in einem Photometer gemessen. Man erhielt die in der nachfolgenden Tabelle aufgeführten Extinktionswerte, die zeigen, daß lösliches Fibrin in Plasma durch die Einwirkung von Thrombin entstand und daß die Menge des löslichen Fibrins von der Dauer der Einwirkung des Thrombins abhängt.

**Tabelle 1**

| Reaktionszeiten in Sekunden | Thrombin-Kinetik (Ext. 492 nm) | phys.NaCl-Kontrolle (Ext. 492 nm) |
|---|---|---|
| 0 | 0,335 | 0,301 |
| 10 | 0,582 | 0,245 |
| 20 | 0,861 | 0,246 |
| 40 | 1,311 | 0,262 |
| 60 | 1,868 | 0,275 |

### Beispiel 5

### Erzeugung von Blutplasma mit definiertem Gehalt an löslichem Fibrin und Bestimmung des Gehalts an Fibrin

5 ml humanes EDTA-Plasma (2 mg/ml EDTA, Tri-Natriumsalz) wurde mit 20 ml physiologischer Kochsalzlösung verdünnt und auf 37°C gebracht. Anschließend wurden 0,167 ml einer Thrombinlösung (30 IU/ml) zugegeben und 30 min bei 37°C inkubiert.

Das Gerinnsel wurde auf Zellstoff abgepreßt und dreimal mit physiologischer Kochsalzlösung gewaschen. Sodann wurde es in 2,5 ml 3 m Harnstofflösung gelöst und die Lösung bei 3000 xg 10 min klarzentrifugiert. Die Messung der Extinktion bei 280 nm ergab unter Berücksichtigung einer spezifischen Extinktion für Fibrin von 15,6 eine Konzentration von 3,1 g/l.

Zur Inaktivierung eventuell noch vorhandenen Thrombins wurde Hirudin (25 µl 1000 IU/ml) zugegeben.

1 ml Human-Citratplasma, enthaltend 2 IU/ml Heparin, wurde mit der oben hergestellten Lösung von Fibrin in Harnstoff auf einen Gehalt von 0,1 mg/ml lösliches Fibrin eingestellt und daraus weitere Verdünnungen in geometrischer Reihe in Heparin-haltigem Citratplasma hergestellt. Die Konzentrationen sind aus Tabelle 2 ersichtlich.

Anschließend wurden Proben wie in Beispiel 4 beschrieben 1:100 in Puffer verdünnt und getestet.

Man erhielt die in Tabelle 2 aufgeführten Extinktionswerte, die in Abhängigkeit von der Menge an löslichem Fibrin zunehmen. Als Kontrollen wurden in Tabelle 2 zusätzlich die mit reiner Harnstofflösung versetzte Citratplasmaprobe sowie eine Probe, in der Puffer anstelle des Plasmas eingesetzt wurde, eingetragen.

**Tabelle 2**

| Probe Nr. | Gehalt an löslichem Fibrin im Plasma (mg/l) | A_{405 nm} |
|---|---|---|
| 1 | 0 | 0,108 |
| 2 | 0,2 | 0,129 |
| 3 | 0,4 | 0,086 |
| 4 | 0,8 | 0,123 |
| 5 | 1,6 | 0,123 |
| 6 | 3,2 | 0,201 |
| 7 | 6,3 | 0,276 |
| 8 | 12,5 | 0,352 |
| 9 | 25,0 | 0,510 |
| 10 | 50,0 | 0,832 |
| 11 | 100,0 | 1,205 |

| Kontrollen | | |
|---|---|---|
| Plasma-Harnstoff | - | 0,102 |
| Puffer | - | 0,002 |

## Patentansprüche

1. Verfahren zur Bestimmung von löslichem Fibrin in einer Körperflüssigkeit einer Spezies, wobei eine Probe dieser Körperflüssigkeit entweder
- mit einem an eine Festphase gebundenen Fibrinogen in Kontakt gebracht wird,
- die Körperflüssigkeit abgetrennt wird,
- die Festphase mit einem markierten Antikörper, der mit Fibrin reagiert, in Kontakt gebracht wird,
- der Überschuß dieses Antikörpers abgetrennt wird und aus der Menge der an die Festphase gebundenen Markierung die Menge "löslichen" Fibrins bestimmt wird
oder
- mit einem an eine Festphase gebundenen Antikörper, der mit Fibrin reagiert, in Kontakt gebracht wird,
- die Körperflüssigkeit abgetrennt wird,
- die Festphase mit einem markierten Fibrinogen in Kontakt gebracht wird,
- der Überschuß dieses Fibrinogens abgetrennt wird und aus der Menge der an die Festphase gebundenen Markierung die Menge "löslichen" Fibrins bestimmt wird
dadurch gekennzeichnet, daß das Fibrinogen und der Antikörper aus der gleichen Tierspezies gewonnen wurden, die verschieden ist von der Spezies von der die Körperflüssigkeit stammt.

2. Verfahren nach Anspruch 1 dadurch gekennzeichnet, daß die Körperflüssigkeit von der Spezies Mensch ist.

3. Verfahren nach Anspruch 1, wobei die Körperflüssigkeit in Gegenwart von zusätzlichem Fibrinogen der gleichen Spezies, wie der von der das festphasengebundene Fibrinogen stammt, mit der Festphase in Kontakt gebracht wird.

4. Verfahren nach Anspruch 1 dadurch gekennzeichnet, daß die Markierung ein Enzym ist.

5. Verwendung von Fibrinogen einer Tierspezies zur Bestimmung von "löslichem" Fibrin in einer Körperflüssigkeit einer anderen Tierspezies in einem Verfahren gemäß Anspruch 1.

## Claims

1. A method for the determination of soluble fibrin in a body fluid of a species, where a sample of this body fluid is either
- contacted with a fibrinogen bound to a solid phase,
- the body fluid is separated off,
- the solid phase is contacted with a labeled antibody which reacts with fibrin,
- the excess of this antibody is separated off, and the amount of "soluble" fibrin is determined from the amount of the label bound to the solid phase,
or
- contacted with an antibody which reacts with fibrin and is bound to a solid phase,
- the body fluid is separated off,
- the solid phase is contacted with a labeled fibrinogen,
- the excess of this fibrinogen is separated off, and the amount of "soluble" fibrin is determined from the amount of the label bound to the solid phase, wherein the fibrinogen and the antibody have been obtained from the same animal species which differs from the species from which the body fluid originates.

2. The method as claimed in claim 1, wherein the species providing the body fluid is man.

3. The method as claimed in claim 1, wherein the body fluid is contacted with the solid phase in the presence of additional fibrinogen from the same species as that from which the fibrinogen bound to the solid phase originates.

4. The method as claimed in claim 1, wherein the label is an enzyme.

5. The use of fibrinogen from one animal species for the determination of "soluble" fibrinogen in a body fluid from another animal species in a method as claimed in claim 1.

## Revendications

1. Procédé pour la détermination de la fibrine soluble dans un liquide corporel d'une espèce, dans lequel un échantillon de ce liquide corporel soit
- est mis en contact avec un fibrinogène lié à une phase solide,
- le liquide corporel est séparé,
- la phase solide est mise en contact avec un anticorps marqué qui réagit avec la fibrine,
- l'excès de cet anticorps est séparé et la quantité de fibrine "soluble" est déterminée à partir de la quantité du marqueur lié à la phase solide,
soit
- est mis en contact avec un anticorps lié à une phase solide, qui réagit avec la fibrine,
- le liquide corporel est séparé,
- la phase solide est mise en contact avec un fibrinogène marqué,
- l'excès de ce fibrinogène est séparé et la quantité de fibrine "soluble" est déterminée à partir de la quantité du marqueur lié à la phase solide,
caractérisé en ce que le fibrinogène et l'anticorps ont été obtenus à partir de la même espèce animale, qui est différente de l'espèce de laquelle provient le liquide corporel.

2. Procédé selon la revendication 1, caractérisé en ce que le liquide corporel est de l'espèce humaine.

3. Procédé selon la revendication 1, dans lequel le liquide corporel est mis en contact avec la phase solide en présence de fibrinogène supplémentaire de la même espèce que celle de laquelle provient le fibrinogène lié à la phase solide.

4. Procédé selon la revendication 1, caractérisé en ce que le marqueur est une enzyme.

5. Utilisation d'un fibrinogène d'une espèce animale pour la détermination de la fibrine "soluble" dans un liquide corporel d'une autre espèce animale, dans un procédé selon la revendication 1.
